# EUROPÄISCHE PATENTSCHRIFT

(11) **EP 1 146 923 B1**
(45) Veröffentlichungstag und Bekanntmachung des Hinweises auf die Patenterteilung: **12.05.2004**
(21) Anmeldenummer: 00900059.7
(22) Anmeldetag: 11.01.2000
(51) Int. Cl.: A61M 5/315, A61M 5/50

(54) **VORRICHTUNG ZUR DOSIERTEN VERABREICHUNG EINES INJIZIERBAREN PRODUKTES**
DEVICE FOR DOSING THE ADMINISTRATION OF AN INJECTABLE PRODUCT
DISPOSITIF POUR L'ADMINISTRATION DOSEE D'UN PRODUIT INJECTABLE

(30) Priorität: 12.01.1999 DE 19900827
(43) Veröffentlichungstag der Anmeldung: 24.10.2001
(73) Patentinhaber: Tecpharma Licensing AG, 3401 Burgdorf (CH)
(72) Erfinder: KIRCHHOFER, Fritz, CH-3454 Sumiswald (CH); GURTNER, Thomas, CH-3425 Koppigen (CH)
(86) Internationale Anmeldenummer: PCT/CH2000/000016
(87) Internationale Veröffentlichungsnummer: WO 2000/041752

(56) Entgegenhaltungen:
- WO-A-97/36626
- FR-A- 2 612 782
- US-A- 5 328 476
- US-A- 5 643 214
- US-A- 5 807 346

## Beschreibung

Die Erfindung betrifft eine Vorrichtung zur dosierten Verabreichung eines injizierbaren Produkts.

Eine Vorrichtung, wie die Erfindung sie betrifft, ist beispielsweise aus der WO 97/36626 bekannt. Die Vorrichtung weist ein Gehäuse mit einem Reservoir für das Produkt auf. In dem Reservoir ist ein Kolben aufgenommen, der bei einer Verschiebung in eine Vorschubrichtung auf einen Auslass des Reservoirs zu Produkt aus dem Reservoir verdrängt. Eine Zahnstange, die gegen den Kolben drückt, schiebt den Kolben in Vorschubrichtung. Die Zahnstange ist mit Zahnreihen versehen. Im Gehäuse ist ferner ein Antriebsglied relativ zum Gehäuse in und gegen die Vorschubrichtung verschiebbar aufgenommen, das bei einer Verschiebung in Vorschubrichtung die Zahnstange mitnimmt. Hierfür greift das Antriebsglied mit Mitnehmern in die Zahnreihen der Zahnstange ein. Bei einem Verschieben drückt stets nur einer der Mitnehmer gegen einen Zahnrücken einer Zahnreihe. Zum Einstellen derjenigen Produktmenge, die mit einem Hub verabreicht wird, wird das Antriebsglied in einer vorderen Stellung um eine eingestellte Dosisweglänge manuell gegen die Vorschubrichtung zurückgezogen. Dabei gleiten die Mitnehmer des Antriebsglieds über die Zähne der Zahnreihen und geben dabei elastisch nach. Ein Zurückverschieben der Zahnstange wird durch relativ zum Gehäuse verschiebegesichert aufgenommene Sperrmittel verhindert. Die Sperrmittel wirken mit einer der Zahnreihen der Zahnstange derart zusammen, dass die Sperrmittel eine Verschiebung der Zahnstange gegen die Vorschubrichtung verhindern und durch elastisches Nachgeben eine Verschiebung der Zahnstange in Vorschubrichtung erlauben. Die Sperrmittel greifen bei einer Verschiebung der Zahnstange nicht gleichzeitig mit tiefstem Zahneingriff in Zahnlücken der Zahnreihen. Es greift stets nur eines der Sperrmittel in eine Zahnlücke, während ein anderes gegen eine Zahnflanke drückend elastisch weggebogen wird.

Der in Bezug auf die Genauigkeit und insbesondere die Sicherheit bei der Dosierung und Verabreichung vorteilhafte Wechseleingriff während einer Verschiebung in Vorschubrichtung führt dazu, dass nach einer längeren Zeit der Lagerung, die ab Werk bis zur ersten Benutzung meist mehrere Monate beträgt, aufgrund einer Materialermüdung eines im Lagerungszustand weggebogenen Sperrmittels oder Mitnehmers dieser Vorteil wieder verlorengeht bzw. erst gar nicht zum Tragen kommt.

Die Erfindung hat es sich zur Aufgabe gemacht, bei solch einer Vorrichtung zur dosierten Verabreichung eines injizierbaren Produkts auch nach einer längeren Zeit der Lagerung noch die exakte Dosierung und Verabreichung des Produkts mit höherer Sicherheit zu gewährleisten.

Die Aufgabe wird durch die Gegenstände der unabhängigen Ansprüche gelöst.

Eine Vorrichtung zur dosierten Verabreichung eines injizierbaren Produkts umfasst ein Gehäuse mit einem Reservoir für das Produkt, einen Kolben, der bei einer Verschiebung in eine Vorschubrichtung auf einen Auslass des Reservoirs zu Produkt aus dem Reservoir verdrängt, eine den Kolben in Vorschubrichtung verschiebende Zahnstange mit einer ersten Zahnreihe und einer zweiten Zahnreihe, ein relativ zum Gehäuse in und gegen die Vorschubrichtung verschiebbares Antriebsglied, das bei einer Verschiebung in Vorschubrichtung die Zahnstange mitnimmt, und ein relativ zum Gehäuse verschiebegesichert angeordnetes Sperrmittel, das mit einer der Zahnreihen zusammenwirkt, derart, dass es eine Verschiebung der Zahnstange gegen die Vorschubrichtung verhindert und eine Verschiebung der Zahnstange in Vorschubrichtung erlaubt.

Vorzugsweise sind wenigstens zwei Sperrmittel vorgesehen, die bei einer Verschiebung der Zahnstange nicht gleichzeitig mit tiefstem Eingriff in Zahnlücken der Zahnreihen eingreifen. Es greift stets nur eines der Sperrmittel in eine Zahnlücke ein, während das andere gegen eine Zahnflanke drückend quer zur Vorschubrichtung elastisch weggedrückt wird.

Nach der Erfindung weist zumindest eine der wenigstens zwei Zahnreihen der Zahnstange eine verlängerte Zahnlücke auf, in die das mit dieser Zahnreihe zusammenwirkende Sperrmittel eingreift, wenn die Zahnstange eine Ausgangsstellung vor eine ersten Verabreichung einnimmt. Die Ausgangsstellung nehmen das Antriebsglied und die Zahnstange relativ zueinander nach dem Zusammenbau bis zu einer ersten Verabreichung ein. Es greift somit in der Ausgangsstellung jedes von wenigstens zwei Sperrmitteln in eine Zahnlücke ein. Unter Beibehaltung des für die Verabreichung vorteilhaften Wechseleingriffs wird eine Materialermüdung des Sperrmittels verhindert.

Vorzugsweise sind mit dem Antriebsglied wenigstens zwei Mitnehmer verschiebegesichert verbunden, die je mit einer der Zahnreihen zusammenwirken, derart, dass bei einer Verschiebung des Antriebsglieds in Vorschubrichtung nur einer der wenigstens zwei Mitnehmer in Vorschubrichtung gegen einen Zahn der Zahnstange drückt, während der andere elastisch an einer Zahnflanke nachgibt. Die Mitnehmer erlauben durch elastisches Nachgeben eine Verschiebung des Antriebsglieds gegen die Vorschubrichtung und relativ zu der Zahnstange.

Nach der Erfindung weist zumindest eine der wenigstens zwei Zahnreihen der Zahnstange eine verlängerte Zahnlücke auf, in die der mit dieser Zahnreihe zusammenwirkende Mitnehmer eingreift, wenn die Zahnstange die genannte Ausgangsstellung vor einer ersten Verabreichung einnimmt. Hierdurch wird eine Materialermüdung der Mitnehmer verhindert.

Unter einer verlängerten Zahnlücke wird eine Zahnlücke verstanden, die in Vorschubrichtung gesehen länger ist als die regelmäßig ausgebildeten weiteren Zahnlücken einer Zahnreihe, die nachfolgend als reguläre Zahnlücken bezeichnet werden. Ein Sperrmittel oder Mitnehmer kann in einem längeren Bereich als bei regulären Zahnlücken vorzugsweise soweit in den verlängerten Bereich eingreifen, wie das Sperrmittel in der Sperrstellung oder der Mitnehmer in der Anschlagstellung in eine reguläre Zahnlücke eingreift. Der Eingriff ist jedenfalls tiefer als er es wäre, wenn dem Sperrmittel oder dem Mitnehmer in der Ausgangsstellung eine reguläre Zahnlücke gegenüberliegen würde. Als tiefster Eingriff wird entsprechend ein Eingriff dann bezeichnet, wenn ein Sperrmittel oder Mitnehmer bis in einen Zahngrund ragt oder wenn ein Sperrmittel oder Mitnehmer soweit auf die Zahnstange zuragt wie in einer Sperrstellung oder Anschlagstellung.

Besonders bevorzugt sind für die Ausgangsstellung in den wenigstens zwei Zahnreihen sowohl eine verlängerte Zahnlücke für wenigstens eines von wenigstens zwei Sperrmitteln als auch eine verlängerte Zahnlücke für wenigstens einen von wenigstens zwei Mitnehmern ausgebildet. Die verlängerte Zahnlücke für das Sperrmittel und die verlängerte Zahnlücke für den Mitnehmer können in einer einzigen Zahnreihe ausgebildet sein. Es ist aber auch möglich, die eine verlängerte Zahnlücke in einer Zahnreihe und die andere verlängerte Zahnlücke in der anderen Zahnreihe auszubilden.

Die Zahnreihen der Zahnstange sind vorzusweise sägezahnförmig mit Zähnen, die sich in Vorschubrichtung verjüngen. Vorzugsweise sind das oder die Sperrmittel und der oder die Mitnehmer in ihrer Form angepasst, so dass ein Wegdrücken bei einer Verschiebung der Zahnstange in Vorschubrichtung möglichst wenig durch Reibung erschwert und eine Verschiebung gegen die Vorschubrichtung durch rein formschlüssigen Eingriff sicher blockiert wird. Grundsätzlich können die Zahnreihen jedoch auch eine andere Form aufweisen, solange die beiden Forderungen der Verschiebbarkeit in Vorschubrichtung und sicheren Verhinderung einer Verschiebung gegen die Vorschubrichtung erfüllt werden.

Vorzugsweise sind die Zahnreihen an der Zahnstange einander gegenüberliegend oder auch nebeneinander ausgebildet; sie könnten grundsätzlich jedoch in beliebigen Bereichen der Zahnstange vorgesehen sein mit entsprechendem Höhenversatz der Sperrmittel und/oder der Mitnehmer. Bevorzugt sind jedoch die Mitnehmer untereinander auf gleicher Höhe angeordnet, und es wird der Wechseleingriff durch Versatz der Zahnreihen um einen Bruchteil einer Zahnteilung erreicht. Bevorzugt sind auch die Sperrmittel untereinander auf gleicher Höhe angeordnet.

Die Mitnehmer, wie auch die Sperrmittel, arbeiten in Erfüllung ihrer jeweiligen Funktion auf gleiche Weise, indem sie gegen einen Zahnrücken der Zahnstange auf Anschlag liegen und dadurch die Mitnahme oder Sperrung der Zahnstange bewirken und durch elastisches Nachgeben ein Zurückschieben oder Vorschieben der Zahnstange erlauben. Sie können gleich oder auch unterschiedlich ausgebildet sein. Das elastische Nachgeben wird vorzugsweise durch Abbiegen einer in Verschieberichtung sich erstreckenden Zunge quer zur Verschieberichtung der Zahnstange erreicht. Grundsätzlich wäre es beispielsweise auch möglich einen Nocken querverschiebbar gegen elastische Rückstellkräfte zu lagern.

In einer Ausführungsvariante ist die Zahnstange mit einer dritten Zahnreihe ausgestattet, in die ein drittes Sperrmittel eingreift, wobei auch das dritte Sperrmittel nicht gleichzeitig mit den wenigstens zwei anderen Sperrmitteln in eine Zahnlücke der Zahnstange eingreift. Vorzugsweise ist an der Zahnstange auf gleicher Höhe sogar noch eine vierte Zahnreihe vorgesehen, in die ein viertes Sperrmittel eingreift. Erfindungsgemäß weist auch die dritte Zahnreihe, und im Falle einer vierten Zahnreihe auch die vierte Zahnreihe, eine verlängerte Zahnlücke auf, in die das jeweilige Sperrmittel in der Ausgangsstellung der Zahnstange eingreift.

In einem bevorzugten Ausführungsbeispiel weist das Antriebsglied bei Ausbildung einer dritten Zahnreihe vorzugsweise einen dritten Mitnehmer auf. Auch der dritte Mitnehmer des Antriebsglieds greift nicht gleichzeitig mit den wenigstens zwei anderen Mitnehmern des Antriebsglieds in eine Zahnlücke der Zahnstange so ein, dass er bei einer Verschiebung in Vorschubrichtung gegen einen Zahn der Zahnstange drückt. Der Eingriff der drei Mitnehmer erfolgt alternierend. Bei Ausbildung einer vierten Zahnreihe weist das Antriebsglied vorzugsweise einen vierten Mitnehmer auf.

Es wird somit ein besonders fein abgestimmter Wechseleingriff der Sperrmittel und/oder der Mitnehmer möglich, und dennoch wird einer Materialermüdung aufgrund einer langen Lagerung vorgebeugt.

In einer Ausführungsvariante greifen die wenigstens zwei Sperrmittel in die wenigstens zwei Zahnreihen der Zahnstange ein, während zwei Mitnehmer des Antriebsglieds in zwei andere Zahnreihen der Zahnstange eingreifen. Durch die damit einhergehende, um die Zahnstange herum abwechselnde Anordnung von Mitnehmern und Sperrmitteln ist es möglich, die Länge der Zahnstange und damit die Länge der Vorrichtung zu verkürzen. Die Sperrmittel und die Mitnehmer können auf gleicher Höhe in Bezug auf die Verschubrichtung vorgesehen sein. Ein Wechseleingriff der Sperrmittel untereinander und der Mitnehmer untereinander ist dennoch möglich. Die Anmelderin behält es sich vor, hierauf separaten Schutz mit und ohne die Ausbildung eines verlängerten Zahnfußes anzustreben.

Bevorzugte Ausführungsbeispiele der Erfindung werden nachfolgend anhand von Figuren erläutert. Es zeigen:
- Fig. 1: ein Injektionsgerät mit einer Zahnstange mit vier Zahnreihen und
- Fig. 2 und 3: die Zahnstange mit eingreifenden Sperrmitteln und Mitnehmern im Detail.

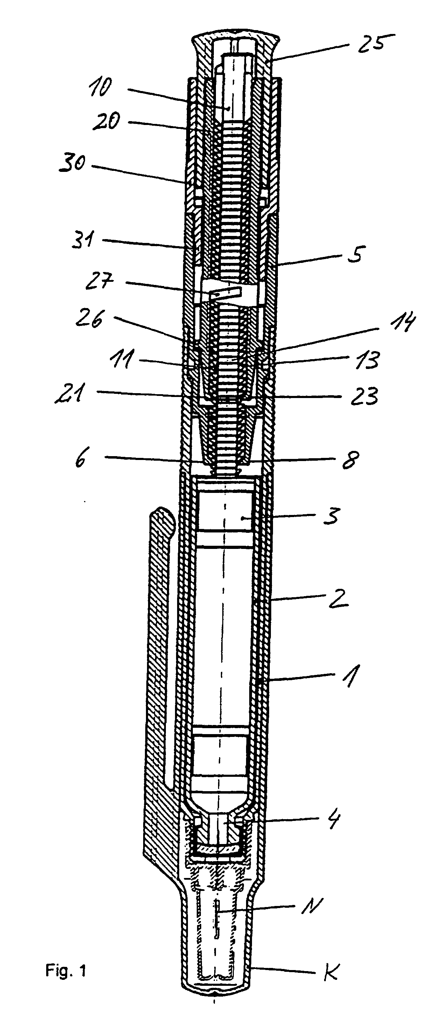

Figur 1 zeigt ein Injektionsgerät, im Ausführungsbeispiel ein Injektionspen, in einem Längsschnitt. Die Figururen 2 und 3 zeigen ein Detail daraus in zwei zueinander senkrechten Längsschnitten.

Das Injektionsgerät weist ein Gehäuse mit einer vorderen Gehäusehülse 1 und einer damit fest verbundenen hinteren Gehäusehülse 5 auf. Die vordere Gehäusehülse 1 dient als Aufnahme für eine Ampulle 2. In der Ampulle 2 ist ein flüssiges Produkt in Form einer Wirkstofflösung, beispielsweise Insulin, enthalten. Ferner ist in der Ampulle 2 ein Kolben 3 aufgenommen. Durch Verschiebung des Kolbens 3 in Vorschubrichtung auf einen Ampullenauslass 4 zu wird das Produkt aus der Ampulle 2 durch deren Auslass 4 hindurch verdrängt und durch eine Injektionsnadel N ausgeschüttet. Die vordere Gehäusehülse 1 ist durch eine Kappe K geschützt. Die Nadel N ist durch eine Nadelkappe nochmals geschützt.

Die Verschiebung des Kolbens 3 in Vorschubrichtung wird durch eine Antriebseinrichtung bewirkt, die in der hinteren Gehäusehülse 5 aufgenommen ist. Die Antriebseinrichtung umfasst als Abtriebsglied eine Zahnstange 10, die unmittelbar auf den Kolben 3 wirkt, und ein Antriebsglied 20. Das Antriebsglied 20 ist in der hinteren Gehäusehülse 5 in und gegen die Vorschubrichtung des Kolbens 3 geradverschiebbar gelagert. Ein Deckel 25, der mit dem Antriebsglied 20 verschiebegesichert verbunden ist, ragt aus dem Gehäuse nach hinten hinaus.

Ein als Hülsenkörper ausgebildetes Dosierglied 30 ist mit der hinteren Gehäusehülse 5 verschiebegesichert, jedoch um die gemeinsame Längsachse verdrehbar verbunden. Durch Verdrehen des Dosierglieds 30 wird die in Vorschubrichtung von dem Antriebsglied 20 und der Zahnstange 10 maximal zurücklegbare Dosisweglänge eingestellt und damit auch die bei einer Injektion maximal ausschüttbare Produktdosis. Hierfür ist ein vorderer Hülsenteil 31 des Dosierglieds 30 an seiner vorderen Stirnfläche spiralig umlaufend ausgebildet, d. h. der vordere Hülsenteil 31 fällt in Bezug auf die Längsachse des Injektionsgeräts von einem vordersten Stimflächenabschnitt in eine Umfangsrichtung fortschreitend ab. Das Dosierglied 30 kann beispielsweise entsprechend einem in der WO 97/36625 beschriebenen Dosierglied ausgebildet sein und bei der Dosierung mit dem Antriebsglied 20 wie dort beschrieben zusammenwirken.

Die Dosierung erfolgt in einer in Bezug auf die Vorschubrichtung vordersten Stellung des Antriebsglieds 20, in der ein von der äußeren Mantelfläche des Antriebsgliebs 20 radial abstehender Kragen bzw. Nocken 26 an einem durch die hintere Gehäusehülse 5 gebildeten Anschlag anliegt. In dieser vordersten Stellung des Antriebsglieds 20 wird das Dosierglied 30 relativ zur hinteren Gehäusehülse 5 verdreht, bis es die gewünschte Dosierstellung erreicht hat. In dieser Dosierstellung verbleibt zwischen einem ebenfalls von der äußeren Mantelfläche des Antriebsglieds 20 abragenden weiteren Kragen bzw. Nocken 27 und der diesem Nocken 27 gegenüberliegenden Stirnfläche des Dosierglieds 30 ein lichter Dosierabstand. Um den Dosierabstand kann das Antriebsglied 20 relativ zur hinteren Gehäusehülse 5 und damit auch relativ zum Kolben 3 gegen die Vorschubrichtung zurückgezogen werden. Das Zurückziehen erfolgt manuell durch Ziehen an dem Deckel 25. Der Dosierabstand ist gleich der Dosisweglänge bei der nachfolgenden Verabreichung.

Bei einem Zurückverschieben bzw. Zurückziehen des Antriebsglieds 20 verbleibt die Zahnstange 10 in ihrer bei dem Dosiervorgang eingenommenen Verschiebelage relativ zum Gehäuse. Sie wird durch an der hinteren Gehäusehülse 5 ausgebildete Sperrmittel 6 und 8 gegen eine Verschiebung gegen die Vorschubrichtung gesichert. Die Sperrmittel 6 und 8 sind Rastnocken, die je an einem vorderen Ende einer elastisch nachgiebigen Zunge ausgebildet sind und von ihrer Zunge radial nach innen auf die Zahnstange 10 ragen. Die Sperrmittel 6 und 8 wirken je mit einer ihnen zugewandten Zahnreihe der Zahnstange 10 zusammen, derart, dass sie eine Verschiebung der Zahnstange 10 in Vorschubrichtung zulassen und eine Verschiebung gegen die Vorschubrichtung durch formschlüssigen Sperreingriff verhindern.

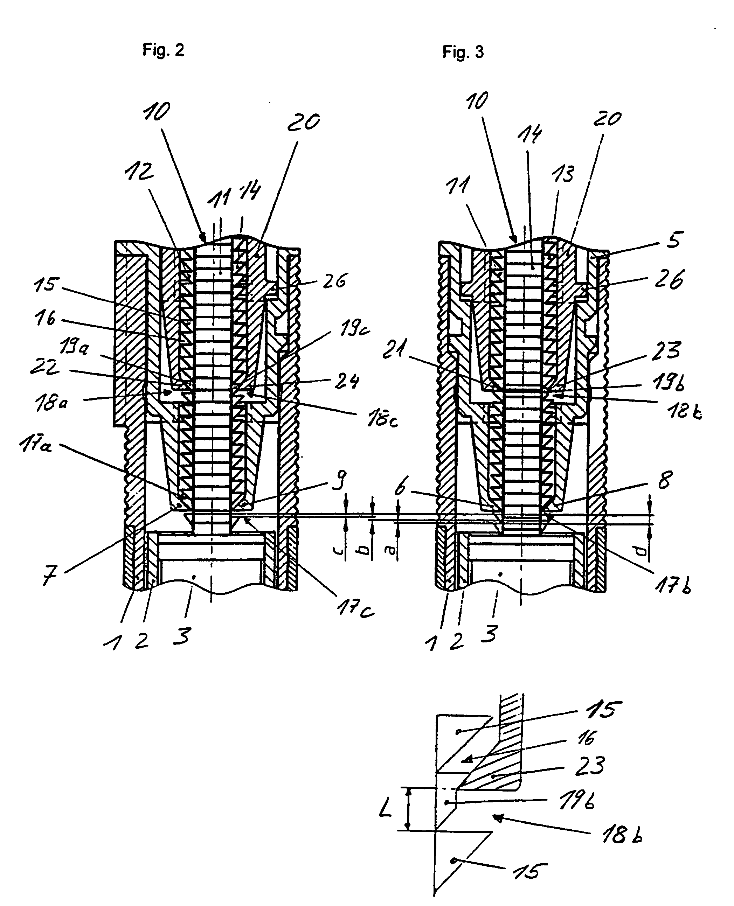

Die Zahnstange 10 wird durch einen im Querschnitt rechteckigen Stangenkörper gebildet, der in einem in Bezug auf die Vorschubrichtung vorderen Bereich an allen vier Seiten mit je einer Sägezahnreihe versehen ist. In Figur 3 sind zwei an gegenüberliegenden Seiten der Zahnstange 10, den Sperrmitteln 6 und 8 gegenüberliegend ausgebildete Zahnreihen mit 11 und 13 bezeichnet. Zusätzlich zu den beiden Zahnreihen 11 und 13 weist die Zahnstange 10 zwei weitere, an gegenüberliegenden Seitenflächen der Zahnstange 10 ausgebildete Sägezahnreihen auf, von denen in Figur 1 die eine mit 14 bezeichnet ist. Die einzelnen Zähne 15 jeder der Zahnreihen der Zahnstange 10 sind jeweils in Vorschubrichtung verjüngt ausgebildet; im Ausführungsbeispiel sind die Zahnflanken einfach plan und schräg. Der Rücken jedes Zahns 15 ist einfach plan und weist senkrecht zur Vorschubrichtung und damit zur Längsrichtung des Injektionsgeräts und der Zahnstange 10. Mit 16 sind jeweils die regelmäßigen bzw. regulären Zahnlücken der Zahnreihen bezeichnet.

Die vier Zahnreihen weisen die gleiche Zahnteilung auf. Sie sind innerhalb einer Zahnteilung zueinander in einem Versatz in Bezug auf die Vorschubrichtung angeordnet. Der Versatz von Zahnreihe zu Zahnreihe ist in den Figuren 2 und 3 mit a, b und c eingetragen.

Die Sperrmittel 6 und 8 und zwei weitere Sperrmittel 7 und 9, die mit den jeweils zugewandten weiteren Zahnreihen 12 und 14 zusammenwirken, befinden sich in Bezug auf die Vorschubrichtung auf gleicher Höhe in jeweils 90° Winkelabstand. Wegen des Zahnreihenversatzes greift stets nur eines der Sperrmittel 6 bis 9 mit einem tiefsten Zahneingriff in eine Zahnlücke 16 der ihm zugewandten Zahnreihe, wenn die Zahnstange 10 vorgeschoben wird. Den drei anderen Sperrmitteln liegen jeweils Flanken von Zähnen 15 der ihnen zugewandten Zahnreihen gegenüber, so dass diese anderen Sperrmittel von der Zahnstange 10 weggebogen werden. Bei einer Verschiebung der Zahnstange 10 in Vorschubrichtung kommen auf diese Weise die Sperrmittel 6 bis 9 sukzessive in tiefstmöglichen Eingriff mit der ihnen jeweils zugewandten Zahnreihe; es ergibt sich insgesamt ein alternierender Eingriff der Sperrmittel. Das jeweils in einen Zahngrund oder zu einem Zahngrund hin elastisch voll eingeschnappte Sperrmittel, sperrt die Zahnstange 10 gegen eine Verschiebung gegen die Vorschubrichtung.

Die Verschiebung der Zahnstange 10 in Vorschubrichtung wird von dem Antriebsglied 20 bewirkt. Hierfür läuft das Antriebsglied 20 in Vorschubrichtung in vier Zungen aus, die an ihren vorderen Enden radial nach innen abragende Rastnocken tragen. Von den derart gebildeten Mitnehmern sind die beiden sich gegenüberliegenden Mitnehmer 21 und 23 in Figur 1 dargestellt. Im Ausführungsbeispiel sind die Mitnehmer und die Sperrmittel in ihrer Form und Funktionsweise gleich. Beide werden durch Rastnocken an elastisch nachgiebigen Zungen gebildet. Bei einer Verschiebung des Antriebsglieds 20 in Vorschubrichtung stemmt sich jeweils einer der Mitnehmer gegen den Rücken eines der Zähne 15 der ihm zugewandten Zahnreihe und bewirkt so die zwangsweise Mitnahme der Zahnstange 10 in Vorschubrichtung. Aufgrund ihrer elastischen Nachgiebigkeit und der Vorwärtspfeilung der Zähne 15 gleiten die Mitnehmer bei einer Verschiebung des Antriebsglieds 20 gegen die Vorschubrichtung über die Zahnreihen der durch die Sperrmittel gesperrten Zahnstange 10. Da die Mitnehmer auf gleicher Höhe in Bezug auf die Vorschubrichtung in Rastnocken auslaufen, greifen auch niemals zwei der Mitnehmer mit tiefstem Zahneingriff gleichzeitig in eine der regulären Zahnlücken 16 der Zahnstange 10 ein.

In den Figuren 1 bis 3 ist das Injektionsgerät in einer Ausgangsstellung dargestellt, in der die Zahnstange 10 ihre hinterste Stellung relativ zu der hinteren Gehäusehülse 5 und auch relativ zum Antriebsglied 20 einnimmt. In dieser Ausgangsstellung wird die hintere Gehäusehälfte 5 komplett montiert mit Zahnstange 10 und Antriebsglied 20 einschließlich Deckel 25 und Dosierglied 30 herstellerseitig geliefert. Die Ausgangsstellung entspricht somit der Lagerstellung des Injektionsgeräts, insbesondere der Antriebs- und Dosiereinrichtung des Injektionsgeräts. Im Ausführungsbeispiel ist das Injektionsgerät ein Einwegpen. Eine Wiederverwendbarkeit, d.h. ein Ampullenaustausch, kann jedoch mit einfachen Modifikationen erreicht werden.

In der Ausgangsstellung des Injektionsgeräts mit eingesetzter Ampulle 2 wird die mit der ersten Injektion zu verabreichende Produktdosis vom Benutzer eingestellt. Hierzu wird das Dosierglied 30 in eine bestimmte Drehstellung gedreht, die der gewünschten Produktdosis entspricht. In dieser Verdrehstellung weist der Nocken 27 des Antriebsglieds 20 zu der ihm gegenüberliegenden vorderen Stirnfläche des Dosierglieds 30 den lichten Dosierabstand auf. Nur das Sperrmittel 6 liegt in der Ausgangsstellung an einem Zahnrücken der Zahnreihe 11 auf Sperranschlag. Die anderen Sperrmittel 7,8 und 9 sind zwar bis in ihre entlasteten Neutralstellungen zur Zahnstange 10 hin vorgeschnappt, sie kommen in der Ausgangsstellung jedoch in Zahnlücken 17a, 17b und 17c zu liegen, die gegenüber den regulären Zahnlücken 16 verlängert sind. Von den Mitnehmern liegt in der Ausgangsstellung nur der Mitnehmer 21 auf Anschlag zu einem Zahnrücken. Die anderen Mitnehmer 22, 23 und 24 liegen in der Ausgangsstellung entlastet in ihren Neutralstellungen in den ihnen zugewandten Zahnlücken, d.h. sie werden in der Ausgangsstellung nicht weggebogen. Vor ihren verlängerten Zahnlücken 17a, 17b und 17c weisen die Zahnreihen 12, 13 und 14 je einen Zahn auf. Diese Zähne, die die verlängerten Zahnlücken 17a, 17b und 17c in Vorschubrichtung begrenzen, dienen lediglich einem Funktionstest des Injektionsgeräts. Unmittelbar nach dem Zusammenbau des Geräts wird die Zahnstange 10 durch die konzentrisch zu ihr angeordneten Sperrmittel 6 bis 9 hindurch bis in die Ausgangsstellung gedrückt.

Das Antriebsglied 20 wird durch Ziehen an dem Deckel 25 aus seiner vordersten Stellung in Bezug auf die hintere Gehäusehülse 5 gegen die Vorschubrichtung zurückgezogen. Bei dem Zurückziehen des Antriebsglieds 20 gleiten dessen Mitnehmer 21 bis 24 über die ihnen zugewandten Zahnreihen der Zahnstange 10, die an einer Mitnahme durch das Sperrmittel 6 gehindert wird.

Bei der Injektion werden das Antriebsglied 20 und damit auch die Zahnstange 10 in Vorschubrichtung durch Drücken gegen den Deckel 25 um die Dosisweglänge verschoben. Dabei drückt die Zahnstange 10 den Kolben 3 in der Ampulle 2 auf den Auslass 4 zu, und es wird Produkt ausgeschüttet. In der in den Figuren gezeigten Ausgangsstellung liegt nur der Mitnehmer 21 in Anschlag gegen einen Zahnrücken der Zahnstange 10.

Im Ausführungsbeispiel sind die Mitnehmer des Antriebsglieds 20 in Bezug auf die Vorschubrichtung hinter den Sperrmitteln angeordnet. Die konzentrische Anordnung der Sperrmittel und der Mitnehmer ist so gestaltet, dass sie entsprechend der Zahnform der Zahnreihen der Zahnstange 10 gegen ihre eigenen elastischen Rückstellkräfte radial nach außen von der Zahnstange 10 weggebogen werden können. Im Ausführungsbeispiel liegen jeweils die Sperrmittel unter sich und jeweils die Mitnehmer unter sich auf gleicher Höhe in Bezug auf die Vorschubrichtung, während die Zahnreihen der Zahnstangen 10 solch einen Versatz zueinander aufweisen, dass die regulären Zahnlücken 16 der Zahnreihen auf unterschiedlichen Höhen in Bezug auf die Vorschubrichtung zu liegen kommen. Hierdurch wird bewirkt, dass niemals mehr als ein Sperrmittel bzw. ein Mimehmer in eine der regulären Zahnlücken 16 eingreift. Statt dieser Anordnung können auch die Sperrmittel und auch die Mitnehmer in Bezug auf die Vorschubrichtung entsprechend auf unterschiedlichen Höhen versetzt und die Zahnreihen der Zahnstange 10 auf gleicher Höhe angeordnet sein. Die im Ausführungsbeispiel gewählte Anordnung hat jedoch fertigungstechnische Vorteile.

Aufgrund der Ausstattung der Zahnstange 10 mit vier Zahnreihen könnte bei der Antriebs- und Dosiereinrichtung unter Beibehaltung der Vorteile des Wechseleingriffs Baulänge eingespart werden, indem die Sperrmittel und die Mitnehmer alle auf gleicher Höhe in Bezug auf die Vorschubrichtung angeordnet werden. Dies kann dadurch bewerkstelligt werden, dass zwei Sperrmittel, beispielsweise die Sperrmittel 6 und 8, in zwei der vier Zahnreihen der Zahnstange 10 und auf gleicher Höhe zwei Mitnehmer des Antriebsglieds 20 in die beiden anderen der Zahnreihen eingreifen. Wegen der Ausbildung eines Paars von Sperrmitteln und eines Paars von Mitnehmern kann bei entsprechend versetzter Anordnung der Zahnreihen oder Sperrmittel und Mitnehmer dennoch der Vorteil des alternierenden Eingriffs erhalten bleiben.

In der in den Figuren dargestellten Ausgangsstellung, die insbesondere für die in der hinteren Gehäusehülse 5 aufgenommenen Teile des Injektionsgeräts, nämlich die Zahnstange 10, das Antriebsglied 20 und die Sperrmittel 6 bis 9 auch die Lagerstellung ist, würde die Gefahr einer Materialermüdung bei solchen Sperrmitteln und Mitnehmern bestehen, die in der Ausgangsstellung nicht in Zahnlücken 16 so einschnappen können, dass sie zumindest teilweise oder, wie im Ausführungsbeispiel, vollkommen entlastet sind. Diese Sperrmittel und Mitnehmer wären nämlich in der Ausgangsstellung abgebogen. In der abgebogenen Stellung sind die Mitnehmer und Sperrmittel elastisch vorgespannt. Hält dieser Zustand über längere Zeiten an, so kann ein elastisches Rückbiegen bis in die Funktionsstellung, nämlich der Anschlagstellung gegen einen Zahnrücken, nicht mit der erforderlichen Sicherheit gewährleistet werden.

Die Zahnstange 10 weist jedoch verlängerte Zahnlücken dort auf, wo in der Ausgangsstellung des Injektionsgeräts Sperrmittel und Mitnehmer eingreifen, die in der Ausgangsstellung nicht auf Anschlag zu Zahnrücken der Zahnstange 10 sind.

Die Figuren 2 und 3 werden nachfolgend in der Zusammenschau beschrieben.

Die Zahnstange 10 ist unmittelbar von ihrer dem Kolben 3 zugewandten Stirnfläche aus an allen vier Seiten mit je einer Sägezahnreihe gleicher Form und Zahnteilung versehen. Die Zahnreihen sind umlaufend mit 11, 12, 13 und 14 bezeichnet. Die erste Zahnreihe 11 weist lückenlos in regelmäßiger Zahnteilung aufeinander folgend einzelne Sägezähne 15 auf. In der in den Figuren dargestellten Ausgangsstellung greifen das Sperrmittel 6 und vom Kolben 3 aus gesehen der dahinter angeordnete Mitnehmer 21 in je eine der regulären Zahnlücken 16 derart ein, dass sie in Vorschubrichtung gesehen auf Anschlag an Zahnrücken sind.

Um die Zahnstange 10 umlaufend folgt auf die erste Zahnreihe 11 die in Figur 2 abgebildete zweite Zahnreihe 12. Die zweite Zahnreihe 12 ist von den folgenden Unterschieden abgesehen identisch zur ersten Zahnreihe 11.

Zum einen sind die Zähne 15 der zweiten Zahnreihe 12 um einen Bruchteil einer Zahnteilung, nämlich um die Länge d gegenüber den Zähnen 15 der ersten Zahnreihe 11 in Bezug auf die Vorschubrichtung versetzt entlang der Zahnstange 10 angeordnet. Durch diesen Versatz und die Anordnung der Sperrmittel 6 und 7 auf gleicher Höhe wird erreicht, dass sich stets nur eines der Sperrmittel 6 und 7 in einem tiefsten Zahneingriff befindet, in dem es seine entlastete Neutralstellung einnimmt. Dies gilt jedoch mit einer Ausnahme, die einen zweiten Unterschied zur ersten Zahnreihe 11 begründet. In einem vorderen Bereich der zweiten Zahnreihe 12 ist nämlich eine verlängerte Zahnlücke 17a ausgebildet, in die in der Ausgangsstellung das Sperrmittel 7 eingreift. Auch das Sperrmittel 7, das bei vollkommen regelmäßiger Ausbildung der zweiten Zahnreihe 12 in der Ausgangsstellung von der Zahnstange 10 abgebogen wäre, befindet sich aufgrund der Verlängerung der Zahnlücken 17a über das RegelmaB der zweiten Zahnreihe 12 hinaus in einem tiefsten Zahneingriff. Es findet somit in der Ausgangsstellung eine elastische Verbiegung der den Mitnehmer 7 am vorderen Ende ausbildenden Zunge nicht statt.

Als weiteren Unterschied weist die zweite Zahnreihe 12 vom Kolben 3 aus gesehen hinter der verlängerten Zahnlücke 17a eine weitere verlängerte Zahnlücke 18a auf. In der weiteren verlängerten Zahnlücke 18a kommt in der Ausgangstellung der zweite Mitnehmer 22 des Antriebsglieds 20 unverspannt zu liegen. Die Eingriffsabfolge der Mitnehmer 21 und 22 entspricht derjenigen der Sperrmittel 6 und 7.

Von der ersten Zahnreihe 11 über die zweite Zahnreihe 12 weiter um die Zahnstange 10 umlaufend folgt die dritte Zahnreihe 13, die im Längsschnitt der Figur 3 abgebildet ist. Die dritte Zahnreihe 13 ist mit einer verlängerten Zahnlücke 17b und einer weiteren verlängerten Zahnlücke 18b versehen, in denen entsprechend der Einbaulage in der Ausgangsstellung das dritte Sperrmittel 8 und der dritte Mitnehmer 23 in der jeweiligen Neutralstellung, d. h. unverspannt, eingreifen.

Noch weiter um die Zahnstange 10 umlaufend folgt auf die dritte Zahnreihe 13 eine vierte Zahnreihe 14. Wie die zweite Zahnreihe 12 und die dritte Zahnreihe 13 weist sie eine verlängerte Zahnlücke 17c und eine weitere verlängerte Zahnlücke 18c auf, in die in der Ausgangsstellung das vierte Sperrmittel 9 und der vierte Mitnehmer 24 mit tiefstem Zahneingriff und deshalb ohne Verspannung der sie tragenden Zungen eingreifen.

Die verlängerten Zahnlücken 17a, 17b und 17c sind auf gleicher Höhe angeordnet entsprechend der Anordnung der in sie in der Ausgangsstellung eingreifenden Sperrmittel 7, 8 und 9. Das gleiche gilt für die weiteren verlängerten Zahnlücken 18a, 18b und 18c, die ebenfalls in Bezug auf die Vorschubrichtung an der Zahnstange 10 auf gleicher Höhe vorgesehen sind.

Die verlängerten Zahnlücken 17a und 18a sind unterschiedlich ausgebildet.

Die verlängerte Zahnlücke 17a wird durch Weglassen eines Zahns unmittelbar beim Gießen der Zahnstange 10 oder durch nachträgliche Wegnahme eines ganzen Zahns 15 gebildet.

Die weitere verlängerte Zahnlücke 18a wird durch Wegnahme nur eines Teils oder Formgießens nur eines Teils eines Zahns so gebildet, dass der in die verlängerte Zahnlücke 18a eingreifende zweite Mitnehmer 22 näher bei dem gegen die Vorschubrichtung nächsten Zahnrücken der zweiten Zahnreihe 12 liegt als der erste Mitnehmer 21 bei dem gegen die Vorschubrichtung nächsten Zahnrücken der ersten Zahnreihe 11. Der die verlängerte Zahnlücke 18a in der zweiten Zahnreihe 12 abschließende Zahnrücken ist dem Kolben 3 näher als der Zahnrücken, der in der ersten Zahnreihe 11 in der Ausgangsstellung die Zahnlücke 16 des Eingriffs abschließt. Verlässt das Antriebsglied 20 beim ersten Dosieren die Ausgangsstellung, gelangt somit der zweite Mitnehmer 22 vor dem ersten Mitnehmer 21 in Anschlag zu einem Zahnrücken.

Unter der Figur 3 ist die dritte Zahnreihe 13 im Bereich ihrer verlängerten Zahnlücke 18b im Detail dargestellt. Die verlängerte Zahnlücke 18b wird dadurch gebildet, dass von drei aufeinanderfolgenden Zähnen der mittlere weniger weit von der Zahnstange abragt als die ihn begrenzenden beiden regulären Zähne 16. Der mittlere Zahn ist abgestumpft und mit 19b bezeichnet. Er ist so ausgebildet, dass der Mitnehmer 23 in der Ausgangsstellung eng an einer Flanke des Zahns 19b unter allenfalls geringer Vorspannung oder vorzugsweise ohne Vorspannung anliegt. Auf diese Weise bleibt in der verlängerten Zahnlücke 18b an dem derart ausgebildeten Zahn 19b ein Zahnrücken an einer Höhe der Zahnstange 10 stehen, an der sich bei vollkommen regulären Ausbildung sämtlicher Zähne der Zahnreihe 13 ebenfalls ein Zahnrücken befinden würde. In die verlängerte Zahnlücke 18b kann der Mitnehmer 23 über eine Länge L mit tiefstem Eingriff eingreifen. Die weiteren verlängerten Zahnlücken 18a und 18c für die Mitnehmer 22 und 24 sind ähnlich wie die verlängerte Zahnlücke 18b ausgebildet, wobei allerdings deren Längen L kürzer sind als diejenige der verlängerten Zahnlücke 18b.

Die Sperrmittel 6 bis 9 ragen ein wenig tiefer in die Zahnlücken 16 als die Mitnehmer 21 bis 24, wenn sie vollkommen auf die Zahnstange 10 zu vorgeschnappt sind.

Es könnten die verlängerten Zahnlücken 18a, 18b und 18c ebenso durch einfaches Weglassen eines Zahns ausgebildet sein, d.h. sie könnten wie die verlängerten Zahnlücken für die Sperrmittel ausgebildet sein. Es könnten die verlängerten Zanlücken 17a, 17b und 17c durch Stehenlassen eines Zahnstumpfs, vorzugsweise in der Art der verlängerten Zahnlücken 18a, 18b und 18c, ausgebildet sein. Eine Vertauschung der Ausbildung wäre ebenso möglich. Die im Ausführungsbeispiel gezeigte Ausbildung der verlängerten Zahnlücke für die Mitnehmer einerseits und die Sperrmittel andererseits ist jedoch die bevorzugte.

In der Ausgangsstellung blockiert das erste Sperrmittel 6 die Zahnstange 10 gegen eine Verschiebung gegen die Vorschubrichtung. In dieser Ausgangsstellung wird zunächst die mit der nächsten Injektion zu verabreichende Produktdosis mit dem Dosierglied 30 Figur 1 gewählt. Anschließend wird das Antriebsglied 20 um den dieser Dosis entsprechende Dosierabstand zurückgezogen. Dabei gleiten die Mitnehmer 21 bis 24 über die Zähne 15 der ihnen jeweils zugewandten Zahnreihe, wobei durch den Versatz der Zahnreihen sichergestellt wird, dass die Mitnehmer 21 bis 24 sukzessive in einem regelmäßigen Wechsel einschnappen, wodurch gegenüber nur einem einzigen Mitnehmer innerhalb einer Zahnteilung mehrere Rastvorgänge stattfinden. In der vom Dosierglied 30 vorgegebenen hintersten Stellung des Antriebsglieds 20 wird ein Einschnappen wenigstens eines der Mitnehmer 21 bis 24 weit sicherer gewährleistet als dies bei nur einer Zahnreihe und einem Mitnehmer der Fall wäre. Sinngemäß das gleiche gilt für das Zusammenwirken der Zahnreihen und der Sperrmittel 6 bis 9. Bei einer Verschiebung des Antriebsglieds 20 gegen die Vorschubrichtung und auch bei einer Verschiebung der Zahnstange 10 in Vorschubrichtung gelangen einer der Mitnehmer und eines der Sperrmittel je als nächstes in tiefsten Zahneingriff und somit in Mitnahmeeingriff bzw. Sperreingriff, die in der Ausgangsstellung je in eine verlängerte Zahnlücke einschnappen. Unmittelbar aus der Ausgangsstellung ist dies der Mitnehmer 23, der mit der dritten Zahnreihe 13 zusammenwirkt. Aufgrund der einfachen Ausbildung der Zahnlücken 17a, 17b und 17c gelangt bei den Sperrmitteln das Sperrmittel 6 als nächstes in Sperreingriff beim Vorschieben der Zahnstange 10 bei einer ersten Verabreichung.

## Patentansprüche

1. Vorrichtung zur dosierten Verabreichung eines injizierbaren Produkts, umfassend
a) ein Gehäuse (1, 5) mit einem Reservoir (2) für das Produkt,
b) einen Kolben (3), der bei einer Verschiebung in eine Vorschubrichtung auf einen Auslass (4) des Reservoirs (2) zu Produkt aus dem Reservoir (2) verdrängt,
c) eine den Kolben (3) in Vorschubrichtung verschiebende Zahnstange (10) mit wenigstens einer ersten Zahnreihe (11) und einer zweiten Zahnreihe (12),
d) ein relativ zum Gehäuse (1, 5) in und gegen die Vorschubrichtung verschiebbares Antriebsglied (20), das bei einer Verschiebung in Vorschubrichtung die Zahnstange (10) mitnimmt, und
e) wenigstens zwei relativ zum Gehäuse (1, 5) verschiebegesichert angeordnete Sperrmittel (6, 7), die je mit einer der Zahnreihen (11, 12) zusammenwirken. derart, dass die Sperrmittel (6, 7) eine Verschiebung der Zahnstange (10) gegen die Vorschubrichtung verhindern und durch elastisches Nachgeben eine Verschiebung der Zahnstange (10) in Vorschubrichtung erlauben, **dadurch gekennzeichnet daß** die Sperrmittel (6, 7) bei einer Verschiebung der Zahnstange (10) nicht gleichzeitig mit einem tiefsten Eingriff in Zahnlücken (16) der Zahnreihen (11, 12) eingreifen, und, dass
f) die zweite Zahnreihe (12) eine verlängerte Zahnlücke (17a) aufweist, in die das mit der zweiten Zahnreihe (12) zusammenwirkende Sperrmittel (7) eingreift, wenn die Zahnstange (10) eine Ausgangsstellung vor einer ersten Verabreichung einnimmt.

2. Vorrichtung nach Anspruch 1, **dadurch gekennzeichnet, dass** eine von dem Kolben (3) aus gesehen in der Zahnreihe (12) unmittelbar hinter der verlängerten Zahnlücke (17a) angeordnete Zahnlücke die nächste Zahnlücke der wenigstens zwei Zahnreihen (11, 12) ist, in die eines der wenigstens zwei Spemnittel (6, 7) eingreift.

3. Vorrichtung nach Anspruch 1 oder 2, **dadurch gekennzeichnet, dass** die Zahnstange (10) eine dritte Zahnreihe (13) aufweist, in die ein drittes Sperrmittel (8) eingreift und dass auch das dritte Sperrmittel (8) bei einer Verschiebung der Zahnstange (10) nicht gleichzeitig mit den wenigstens zwei anderen Sperrmitteln (6, 7) mit einem tiefsten Zahneingriff in eine Zahnlücke (16) der Zahnstange (10) eingreift.

4. Vorrichtung nach dem vorhergehenden Anspruch, **dadurch gekennzeichnet, dass** auch die dritte Zahnreihe (13) eine verlängerte Zahnlücke (17b) aufweist, in die das mit der dritten Zahnreihe (13) zusammenwirkende dritte Sperrmittel (8) mit tiefstem Zahneingriff eingreift, wenn die Zahnstange (10) eine Ausgangsstellung vor einer ersten Verabreichung einnimmt.

5. Vorrichtung nach Anspruch 3 oder 4, **dadurch gekennzeichnet, dass** die Zahnstange (10) eine vierte Zahnreihe (14) aufweist, in die ein viertes Sperrmittel (9) eingreift und dass auch das vierte Sperrmittel (9) bei einer Verschiebung der Zahnstange (10) nicht gleichzeitig mit den wenigstens zwei anderen Sperrmitteln (6, 7) und vorzugsweise auch nicht gleichzeitig mit dem dritten Sperrmittel (8) mit einem tiefsten Zahneingriff in eine Zahnlücke (16) der Zahnstange (10) eingreift.

6. Vorrichtung nach dem vorhergehenden Anspruch, **dadurch gekennzeichnet, dass** auch die vierte Zahnreihe (14) eine verlängerte Zahnlücke (17c) aufweist, in die das mit der vierten Zahnreihe (14) zusammenwirkende vierte Sperrmittel (9) mit tiefstem Zahneingriff eingreift, wenn die Zahnstange (10) eine Ausgangsstellung vor einer ersten Verabreichung einnimmt.

7. Vorrichtung zur dosierten Verabreichung eines injizierbaren Produkts, umfassend
a) ein Gehäuse (1, 5) mit einem Reservoir (2) für das Produkt,
b) einen Kolben (3), der bei einer Verschiebung in eine Vorschubrichtung auf einen Auslass (4) des Reservoirs (2) zu Produkt aus dem Reservoir (2) verdrängt,
c) eine den Kolben (3) in Vorschubrichtung verschiebende Zahnstange (10) mit wenigstens einer ersten Zahnreihe (11) und einer zweiten Zahnreihe (12),
d) ein relativ zum Gehäuse (1, 5) in und gegen die Vorschubrichtung verschiebbares Antriebsglied (20), mit dem wenigstens zwei Mitnehmer (21, 22) verschiebegesichert verbunden sind, die je mit einer der Zahnreihen (11, 12) zusammenwirken, derart, dass bei einer Verschiebung des Antriebsglieds (20) nur einer der wenigstens zwei Mitnehmer (21, 22) in Vorschubrichtung gegen einen Zahn (15) der Zahnstange (10) drückt, während der andere elastisch an einer Zahnflanke nachgibt, wobei die Mitnehmer (21, 22) durch elastisches Nachgeben eine Verschiebung des Antriebsglieds (20) gegen die Vorschubrichtung und relativ zu der Zahnstange (10) erlauben, und
e) ein relativ zum Gehäuse (1, 5) verschiebegesichert angeordnetes Sperrmittel (6), das mit wenigstens einer der Zahnreihen (11, 12) zusammenwirkt, derart, dass es eine Verschiebung der Zahnstange (10) gegen die Vorschubrichtung verhindert und eine Verschiebung der Zahnstange (10) in Vorschubrichtung erlaubt, und wobei
f) die zweite Zahnreihe (12) eine verlängerte Zahnlücke (19a) aufweist, in die der mit der zweiten Zahnreihe (12) zusammenwirkende Mitnehmer (22) eingreift, wenn die Zahnstange (10) eine Ausgangsstellung vor einer ersten Verabreichung einnimmt.

8. Vorrichtung nach dem vorhergehenden Anspruch, **dadurch gekennzeichnet, dass** eine von dem Kolben (3) aus gesehen in der zweiten Zahnreihe (12) unmittelbar hinter der verlängerten Zahnlücke (18a) angeordnete Zahnlücke (16) die nächste Zahnlücke der wenigstens zwei Zahnreihen (11, 12) ist, in die einer der wenigstens zwei Mitnehmer (21, 22) eingreift.

9. Vorrichtung nach einem der beiden vorhergehenden Ansprüche, **dadurch gekennzeichnet, dass** die Zahnstange (10) mit einer dritten Zahnreihe (13) versehen ist, in die ein dritter Mitnehmer (23) des Antriebsglieds (20) eingreift, derart, dass bei einer Verschiebung des Antriebsglieds (20) nur einer der Mitnehmer (21, 22, 23) in Vorschubrichtung gegen einen Zahn (15) der Zahnstange (10) drückt und die Mitnehmer (21, 22, 23) durch elastisches Nachgeben eine Verschiebung des Antriebsglieds (20) gegen die Vorschubrichtung und relativ zu der Zahnstange (10) erlauben und dass die Zahnstange (10) in der dritten Zahnreihe (13) eine verlängerte Zahnlücke (18b) aufweist, in die der mit der dritten Zahnreihe (13) zusammenwirkende Mitnehmer (23) eingreift, wenn die Zahnstange (10) die Ausgangsstellung einnimmt.

10. Vorrichtung nach dem vorhergehenden Anspruch, **dadurch gekennzeichnet, dass** die Zahnstange (10) mit einer vierten Zahnreihe (14) versehen ist, in die ein vierter Mitnehmer (24) des Antriebsglieds (20) eingreift, derart, dass bei einer Verschiebung des Antriebsglieds (20) nur einer der Mitnehmer (21, 22, 23, 24) in Vorschubrichtung gegen einen Zahn (15) der Zahnstange (10) drückt und die Mitnehmer (21, 22, 23, 24) durch elastisches Nachgeben eine Verschiebung des Antriebsglieds (20) gegen die Vorschubrichtung und relativ zu der Zahnstange (10) erlauben und dass die Zahnstange (10) in der vierten Zahnreihe (14) eine verlängerte Zahnlücke (18c) aufweist, in die der mit der vierten Zahnreihe (14) zusammenwirkende Mitnehmer (24) eingreift, wenn die Zahnstange (10) die Ausgangsstellung einnimmt.

## Claims

1. A device for the metered administration of an injectable product, comprising
a) a housing (1, 5) having a reservoir (2) for the product,
b) a plunger (3) which upon displacement in a feed direction towards an outlet (4) of the reservoir (2) forces product out of the reservoir (2),
c) a rack (10) having at least a first row of teeth (11) and a second row of teeth (12), for displacing the plunger (3) in the feed direction,
d) a drive member (20) which is displaceable relative to the housing (1, 5) in and in opposite relationship to the feed direction and which upon displacement in the feed direction entrains the rack (10), and
e) at least two blocking means (6, 7) which are arranged in such a way as to be prevented from displacement relative to the housing (1, 5) and which each co-operate with a respective one of the rows of teeth (11, 12) in such a way that the blocking means (6, 7) prevent displacement of the rack (10) in opposite relationship to the feed direction and allow displacement of the rack (10) in the feed direction by elastically yielding, **characterised in that** upon displacement of the rack (10) the blocking means (6, 7) do not simultaneously engage with a deepest engagement into gaps (16) between the teeth of the rows of teeth (11, 12), and
f) the second row of teeth (12) has an elongated tooth gap (17a) into which the blocking means (7) co-operating with the second row of teeth (12) engages when the rack (10) assumes a starting position prior to a first administration operation.

2. A device according to claim 1 **characterised in that** a tooth gap arranged in the row of teeth (12) directly after the elongated tooth gap (17a) as viewed from the plunger (3) is the next tooth gap of the at least two rows of teeth (11, 12), into which one of the at least two blocking means (6, 7) engages.

3. A device according to claim 1 or claim 2 **characterised in that** the rack (10) has a third row of teeth (13) into which a third blocking means (8) engages and that upon displacement of the rack (10) the third blocking means (8) also does not engage simultaneously with the at least two other blocking means (6, 7) with a deepest tooth engagement into a gap (16) in the teeth of the rack (10).

4. A device according to the preceding claim **characterised in that** the third row of teeth (13) also has an elongated tooth gap (17b) into which the third blocking means (8) which co-operates with the third row of teeth (13) engages with the deepest tooth engagement when the rack (10) assumes a starting position prior to a first administration operation.

5. A device according to claim 3 or claim 4 **characterised in that** the rack (10) has a fourth row of teeth (14) into which a fourth blocking means (9) engages and that upon displacement of the rack (10) the fourth blocking means (9) also does not engage simultaneously with the at least two other blocking means (6, 7) and preferably also not simultaneously with the third blocking means (9) with a deepest tooth engagement into a gap (16) in the teeth of the rack (10).

6. A device according to the preceding claim **characterised in that** the fourth row of teeth (14) also has an elongated tooth gap (17c) into which the fourth blocking means (9) which co-operates with the fourth row of teeth (14) engages with the deepest tooth engagement when the rack (10) assumes a starting position prior to a first administration operation.

7. A device for the metered administration of an injectable product comprising
a) a housing (1, 5) having a reservoir (2) for the product,
b) a plunger (3) which upon displacement in a feed direction towards an outlet (4) of the reservoir (2) forces product out of the reservoir (2),
c) a rack (10) having at least a first row of teeth (11) and a second row of teeth (12), which displaces the plunger (3) in the feed direction,
d) a drive member (20) which is displaceable relative to the housing (1, 5) in and in opposite relationship to the feed direction and to which at least two entrainment means (21, 22) are connected in such a way as to be prevented from displacement, which entrainment means each co-operate with a respective one of the rows of teeth (11, 12) in such a way that upon displacement of the drive member (20) only one of the at least two entrainment means (21, 22) presses in the feed direction against a tooth (15) of the rack (10) while the other yields elastically at a tooth flank, wherein the entrainment means (21, 22) by elastically yielding permit displacement of the drive member (20) in opposite relationship to the feed direction and relative to the rack (10), and
e) a blocking means (6) which is arranged in such a way as to be prevented from displacement relative to the housing (1, 5) and which co-operates with at least one of the rows of teeth (11, 12) in such a way that it prevents displacement of the rack (10) in opposite relationship to the feed direction and allows displacement of the rack (10) in the feed direction, and wherein
f) the second row of teeth (12) has an elongated tooth gap (19a) into which the entrainment means (22) which co-operates with the second row of teeth (12) engages when the rack (10) assumes a starting position prior to a first administration operation.

8. A device according to the preceding claim **characterised in that** a tooth gap (16) which is arranged immediately after the elongated tooth gap (18a) in the second row of teeth (12) as viewed from the plunger (3) is the next tooth gap of the at least two rows of teeth (11, 12), into which one of the at least two entrainment means (21, 22) engages.

9. A device as set forth in one of the two preceding claims **characterised in that** the rack (10) is provided with a third row of teeth (13) into which a third entrainment means (23) of the drive member (20) engages in such a way that upon displacement of the drive member (20) only one of the entrainment means (21, 22, 23) pushes in the feed direction against a tooth (15) of the rack (10) and the entrainment means (21, 22, 23) by elastically yielding permit a displacement of the drive member (20) in opposite relationship to the feed direction and relative to the rack (10) and that the rack (10) has in the third row of teeth (13) an elongated tooth gap (18b) into which the entrainment means (23) which co-operates with the third row of teeth (13) engages when the rack (10) assumes a starting position.

10. A device according to the preceding claim **characterised in that** the rack (10) is provided with a fourth row of teeth (14) into which a fourth entrainment means (24) of the drive member (20) engages in such a way that upon displacement of the drive member (20) only one of the entrainment means (21, 22, 23, 24) pushes in the feed direction against a tooth (15) of the rack (10) and the entrainment means (21, 22, 23, 24) by elastically yielding permit a displacement of the drive member (20) in opposite relationship to the feed direction and relative to the rack (10) and that the rack (10) has in the fourth row of teeth (14) an elongated tooth gap (18c) into which the entrainment means (24) which co-operates with the fourth row of teeth (14) engages when the rack (10) assumes a starting position.

## Revendications

1. Dispositif pour l'administration dosée d'un produit injectable, comprenant
a) un boîtier (1, 5) avec un réservoir (2) pour le produit,
b) un piston (3) qui refoule du produit hors du réservoir (2) lorsqu'il est déplacé dans une direction d'avancement vers une sortie (4) du réservoir (2),
c) une crémaillère (10) déplaçant le piston (3) en direction d'avancement avec au moins une première rangée de dents (11) et une deuxième rangée de dents (12),
d) un organe d'entraînement (20) déplaçable par rapport au boîtier (1, 5) dans la direction d'avancement et à l'encontre de celle-ci, lequel entraîne la crémaillère (10) lors d'un déplacement en direction d'avancement, et
e) au moins deux organes de blocage (6, 7) agencés de manière verrouillée par rapport au boîtier (1, 5), qui coopèrent chacun avec une des rangées de dents (11, 12) de telle sorte que les organes de blocage (6, 7) empêchent un déplacement de la crémaillère (10) à l'encontre de la direction d'avancement et permettent, par relâchement élastique, un déplacement de la crémaillère (10) en direction d'avancement, **caractérisé en ce que** lors d'un déplacement de la crémaillère (10), les organes de blocage (6, 7) ne s'engagent pas simultanément par un engagement à profondeur maximum dans des entredents (16) des rangées de dents (11, 12), et **en ce que**
f) la deuxième rangée de dents (12) présente un entredent (17a) prolongé dans lequel s'engage l'organe de blocage (7) coopérant avec la deuxième rangée de dents (12) lorsque la crémaillère (10) occupe une position initiale avant une première administration.

2. Dispositif selon la revendication 1, **caractérisé en ce qu'**un entredent agencé dans la rangée de dents (12) directement derrière l'entredent (17a) prolongé, vu depuis le piston (3), est l'entredent suivant desdites au moins deux rangées de dents (11, 12) dans lequel s'engage l'un desdits au moins deux organes de blocage (6, 7).

3. Dispositif selon l'une ou l'autre des revendications 1 et 2, **caractérisé en ce que** la crémaillère (10) présente une troisième rangée de dents (13) dans laquelle s'engage un troisième organe de blocage (8) et **en ce que** lors d'un déplacement de la crémaillère (10), le troisième organe de blocage (8) ne s'engage pas non plus simultanément avec lesdits au moins deux autres organes de blocage (6, 7) dans un entredent (16) de la crémaillère (10) par un engagement de dent à profondeur maximum.

4. Dispositif selon la revendication précédente, **caractérisé en ce que** la troisième rangée de dents (13) présente aussi un entredent (17b) prolongé, dans lequel le troisième organe de blocage (8) coopérant avec la troisième rangée de dents (13) s'engage par engagement de dent à profondeur maximum, lorsque la crémaillère (10) occupe une position initiale avant une première administration.

5. Dispositif selon l'une ou l'autre des revendications 3 et 4, **caractérisé en ce que** la crémaillère (10) présente une quatrième rangée de dents (14) dans laquelle s'engage un quatrième organe de blocage (9) et **en ce que** lors d'un déplacement de la crémaillère (10), le quatrième organe de blocage (9) ne s'engage pas non plus simultanément avec lesdits au moins deux autres organes de blocage (6, 7), et de préférence pas non plus simultanément avec le troisième organe de blocage (8) dans un entredent (16) de la crémaillère (10) par un engagement de dent à profondeur maximum.

6. Dispositif selon la revendication précédente, **caractérisé en ce que** la quatrième rangée de dents (14) présente aussi un entredent (17c) prolongé, dans lequel le quatrième organe de blocage (9) coopérant avec la quatrième rangée de dents (14) s'engage par engagement de dent à profondeur maximum, lorsque la crémaillère (10) occupe une position initiale avant une première administration.

7. Dispositif pour l'administration dosée d'un produit injectable, comprenant
a) un boîtier (1, 5) avec un réservoir (2) pour le produit,
b) un piston (3) qui refoule du produit hors du réservoir (2) lorsqu'il est déplacé dans une direction d'avancement vers une sortie (4) du réservoir (2),
c) une crémaillère (10) déplaçant le piston (3) en direction d'avancement avec au moins une première rangée de dents (11) et une deuxième rangée de dents (12),
d) un organe d'entraînement (20) déplaçable par rapport au boîtier (1, 5) dans la direction d'avancement et à l'encontre de celle-ci, auquel sont reliés de manière verrouillée au moins deux tocs d'entraînement (21, 22) qui coopèrent chacun avec une des rangées de dents (11, 12) de telle sorte que lors d'un déplacement de l'organe d'entraînement (20), l'un seulement desdits au moins deux tocs d'entraînement (21, 22) presse en direction d'avancement contre une dent (15) de la crémaillère (10), tandis que l'autre se relâche élastiquement sur un flanc de dent, les tocs d'entraînement (21, 22) permettant par relâchement élastique un déplacement de l'organe d'entraînement (20) à l'encontre de la direction d'avancement et par rapport à la crémaillère (10), et
e) un organe de blocage (6) agencé de manière verrouillée par rapport au boîtier (1, 5), et qui coopère avec au moins une des rangées de dents (11, 12) de telle sorte qu'il empêche un déplacement de la crémaillère (10) à l'encontre de la direction d'avancement et qui permet un déplacement de la crémaillère (10) en direction d'avancement, et dans lequel
f) la deuxième rangée de dents (12) présente un entredent (19a) prolongé dans lequel s'engage le toc d'entraînement (22) coopérant avec la deuxième rangée de dents (12) lorsque la crémaillère (10) occupe une position initiale avant une première administration.

8. Dispositif selon la revendication précédente, **caractérisé en ce qu'**un entredent (16) agencé dans la deuxième rangée de dents (12) directement derrière l'entredent (18a) prolongé, vu depuis le piston (3), est l'entredent suivant desdites au moins deux rangées de dents (11, 12), dans lequel s'engage l'un desdits au moins deux tocs d'entraînement (21, 22).

9. Dispositif selon l'une des deux revendications précédentes, **caractérisé en ce que** la crémaillère (10) est pourvue d'une troisième rangée de dents (13) dans laquelle s'engage un troisième toc d'entraînement (23) de l'organe d'entraînement (20), de telle sorte que lors d'un déplacement de l'organe d'entraînement (20), l'un seulement des tocs d'entraînement (21, 22, 23) presse en direction d'avancement contre une dent (15) de la crémaillère (10) et les tocs d'entraînement (21, 22, 23) permettent par relâchement élastique un déplacement de l'organe d'entraînement (20) à l'encontre de la direction d'avancement et par rapport à la crémaillère (10), et **en ce que** la crémaillère (10) présente dans la troisième rangée de dents (13) un entredent (18b) prolongé dans lequel s'engage le toc d'entraînement (23) coopérant avec la troisième rangée de dents (13) lorsque la crémaillère (10) occupe la position initiale.

10. Dispositif selon la revendication précédente, **caractérisé en ce que** la crémaillère (10) est pourvue d'une quatrième rangée de dents (14) dans laquelle s'engage un quatrième toc d'entraînement (24) de l'organe d'entraînement (20), de telle sorte que lors d'un déplacement de l'organe d'entraînement (20), l'un seulement des tocs d'entraînement (21, 22, 23, 24) presse en direction d'avancement contre une dent (15) de la crémaillère (10) et les tocs d'entraînement (21, 22, 23, 24) permettent par relâchement élastique un déplacement de l'organe d'entraînement (20) à l'encontre de la direction d'avancement et par rapport à la crémaillère (10), et **en ce que** la crémaillère (10) présente dans la quatrième rangée de dents (14) un entredent (18c) prolongé dans lequel s'engage le toc d'entraînement (24) coopérant avec la quatrième rangée de dents (14) lorsque la crémaillère (10) occupe la position initiale.
